(19) Europäisches Patentamt European Patent Office Office européen des brevets

(11) **EP 3 831 636 B1**

(12) **EUROPEAN PATENT SPECIFICATION**

(45) Date of publication and mention of the grant of the patent:
**22.02.2023 Bulletin 2023/08**

(21) Application number: **21164727.6**

(22) Date of filing: **24.03.2021**

(51) International Patent Classification (IPC):
**G10L 15/00** (2006.01)   **G10L 25/63** (2013.01)
**G16H 20/70** (2018.01)   **G16H 50/20** (2018.01)
**G16H 50/30** (2018.01)   **G06N 3/04** (2006.01)
**G06N 5/00** (2006.01)   **B60K 35/00** (2006.01)
**B60K 37/06** (2006.01)

(52) Cooperative Patent Classification (CPC):
**B60K 37/06; B60K 35/00; G16H 20/70;**
**G16H 50/20; G16H 50/30;** B60K 2370/148;
B60K 2370/1575; B60K 2370/164; B60K 2370/166;
B60K 2370/736; B60W 2540/22; B60W 2540/221;
G06N 3/044; G06N 3/045; G06N 5/01;      (Cont.)

(54) **METHOD FOR REGULATING USER EMOTION, DEVICE, AND READABLE STORAGE MEDIUM**

VERFAHREN ZUR REGULIERUNG VON BENUTZEREMOTIONEN, VORRICHTUNG UND LESBARES SPEICHERMEDIUM

PROCÉDÉ DE RÉGULATION DE L'ÉMOTION D'UN UTILISATEUR, DISPOSITIF ET SUPPORT DE STOCKAGE LISIBLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.06.2020 CN 202010518290**

(43) Date of publication of application:
**09.06.2021 Bulletin 2021/23**

(73) Proprietor: **BAIDU ONLINE NETWORK TECHNOLOGY (BEIJING) CO., LTD.**
**Beijing 100085 (CN)**

(72) Inventors:
• **XIA, Deguo**
  **Haidian District, Beijing 100085 (CN)**
• **ZHANG, Liuhui**
  **Haidian District, Beijing 100085 (CN)**
• **HUANG, Jizhou**
  **Haidian District, Beijing 100085 (CN)**

(74) Representative: **Mewburn Ellis LLP**
**Aurora Building**
**Counterslip**
**Bristol BS1 6BX (GB)**

(56) References cited:
**CN-A- 106 803 423     KR-A- 20110 102 058**

(52) Cooperative Patent Classification (CPC): (Cont.)
     G06N 20/10; G06N 20/20; G10L 15/00; G10L 25/63

**Description**

TECHNICAL FIELD

The present invention relates to the computer technology, and specifically to the field of natural language understanding and intelligent driving technology.

BACKGROUND

With the rapid development of cities and the increase of human activity areas, more and more users begin to travel by car. During driving, users may feel tired, anxious and other negative emotions if they encounter a traffic jam, wait for a long time, or the like, which is likely to cause traffic hazards.

At present, some smart vehicle-mounted devices are configured to have functionalities such as a music playback functionality, to regulate a bad emotion of a user. However, each user has different personal characteristics, and the degree of acceptance of the each user for an emotion regulation mode varies, which makes it difficult for an existing emotion regulation mode to effectively regulate the emotion of the user, and thus, it is difficult to reduce risks during the driving.

KR20110102058A provides a method for providing emotional therapy in vehicles, where the user requests sensibility treatment execution, and the control module outputs and displays a plurality of sensibility treatment modes, so that when the user selects a desired sensibility treatment mode, a sound source or other manners are called to execute the desired sensibility treatment mode.

CN 106803423 A provides an intelligent system which plays appropriate music or modify the voice output by the navigation device to adjust the user mood.

SUMMARY

Aspects of the invention are set out in the appended claims.

According to the technique of the present invention, the emotion of the user can be effectively regulated.

It should be understood that the content described in this section is not intended to identify key or important features of the embodiments of the present invention, and is not used to limit the scope of the present invention. Other features of the present invention will be easily understood through the following description.

BRIEF DESCRIPTION OF THE DRAWINGS

Accompanying drawings are used for a better understanding of the scheme, and do not constitute a limitation to the present invention.

Fig. 1 is a flowchart of a first method for regulating a user emotion in an embodiment of the present invention;

Fig. 2 is a flowchart of a second method for regulating a user emotion in an embodiment of the present invention;

Fig. 3A is a flowchart of a third method for regulating a user emotion in an embodiment of the present invention;

Fig. 3B is a schematic diagram of an interface of an electronic map in an embodiment of the present invention;

Fig. 4 is a structural diagram of an apparatus for regulating a user emotion in an embodiment of the present invention; and

Fig. 5 is a block diagram of an electronic device adapted to implement the method for regulating a user emotion according to embodiments of the present invention.

DETAILED DESCRIPTION OF EMBODIMENTS

Example embodiments of the present invention are explained below in combination with accompanying drawings, and various details of embodiments of the present invention are included in the explanation to facilitate understanding, and should be regarded as merely as examples. Therefore, it should be recognized by those of ordinary skill in the art that various changes and modifications may be made to the embodiments described herein without departing from the scope of the present invention. Likewise, for clarity and conciseness, descriptions for well-known functions and structures are omitted in the following description.

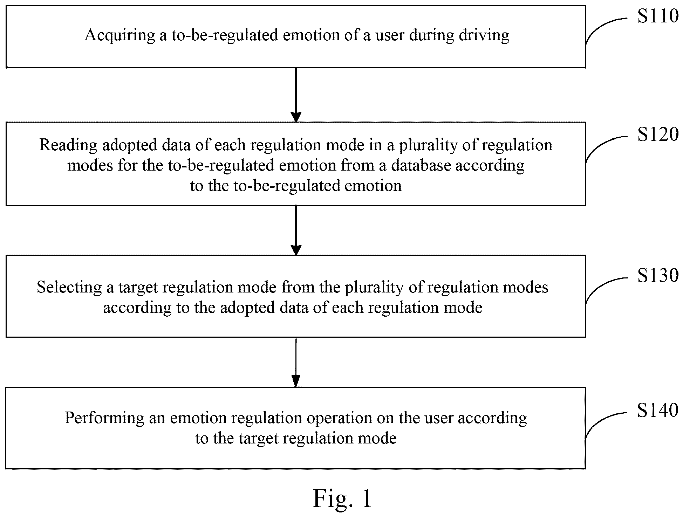

According to an embodiment of the present invention, Fig. 1 is a flowchart of a first method for regulating a user emotion in the embodiment of the present invention. The embodiment of the present invention is applicable to a situation where a regulation mode is selected to automatically regulate an emotion of a user in a driving scenario. The method is performed by an apparatus for regulating a user emotion, and the apparatus is implemented by means of software and/or hardware, and specifically configured in an electronic device having a certain data computing capability. The electronic device may be a vehicle-mounted terminal or a portable smart device. Here, the portable smart device includes, but is not limited to, a smartphone, a smart bracelet, smart eyeglasses, and the like.

The method for regulating a user emotion shown in Fig. 1 includes the following steps.

S 110, acquiring a to-be-regulated emotion of a user during driving.

The user may be the driver in a vehicle. During the driving, the user may have various emotions due to a road condition such as a waiting for traffic lights or a traffic jam or a personal reason. As an example, the user may have a negative emotion such as depression, sadness, anger or the like. As another example, the user may have a positive emotion such as happiness, joy or the like. The to-be-regulated emotion in this embodiment is mainly a negative emotion.

Alternatively, the electronic device collects the physiological data of the user, and analyzes the physiological data to obtain the to-be-regulated emotion of the user. The physiological data includes, but not limited to, data that can reflect the emotion of the user, for example, voice, a facial image, and the grip strength on a steering wheel.

S 120, reading adopted data of each regulation mode in a plurality of regulation modes for the to-be-regulated emotion from a database according to the to-be-regulated emotion.

The database may be configured in the electronic device or in a server remotely connected to the electronic device. The database pre-stores a plurality of regulation modes for each emotion and adopted data of each regulation mode.

Here, the regulation mode is a smart regulation mode that the electronic device can provide, for example, playing music, broadcasting a joke, playing a video, providing a position of an entertainment place or a leisure place near the current position of the vehicle, and the electronic device may further automatically navigating to the position. The regulation mode is not specifically defined in this embodiment. Alternatively, the regulation mode for each emotion may be the same or different. In an alternative implementation, all regulation modes may regulate each emotion.

Since the personal characteristic of each user is different and the degree of acceptance of each user for the regulation mode is different, the adopted data of each regulation mode for each emotion is different. The adopted data in this embodiment may be the data of a regulation mode adopted by a current user, or may be the data of a regulation mode adopted by a user group.

According to the invention, the adopted data includes at least one of: a number of times that the regulation mode is adopted, a frequency at which the regulation mode is adopted, or a rate at which the regulation mode is adopted. Here, the frequency at which the regulation mode is adopted is a number of times that the regulation mode is adopted in a set duration, and the set duration may be, for example, one month. The rate at which the regulation mode is adopted is a quotient obtained by dividing a number of presentations of the regulation mode by a number of times that the regulation mode is adopted. In this embodiment, the degree of acceptance for the regulation mode is accurately reflected in three dimensions: the number of times that the regulation mode is adopted, the frequency at which the regulation mode is adopted and the rate at which the regulation mode is adopted.

After the to-be-regulated emotion is acquired, the to-be-regulated emotion is compared in the database, and adopted data of the each regulation mode in the plurality of regulation modes for the to-be-regulated emotion is read.

S 130, selecting a target regulation mode from the plurality of regulation modes according to the adopted data of the each regulation mode.

Since the adopted data reflects the degree of acceptance for the regulation mode, a regulation mode with a high degree of acceptance may be selected as the target regulation mode, and a number of target regulation modes is at least one.

In an alternative implementation, a regulation mode to which adopted data exceeding a set threshold value belongs is used as the target regulation mode. The set threshold value may be set autonomously, for example, the set threshold value of the number of adoptions is 100.

In another alternative implementation, the regulation modes to which the adopted data belongs are sorted in descending order of the adopted data. A set number of top-ranked regulation modes are determined as target regulation modes. The set number may be 1, 2 or 3.

S140, performing an emotion regulation operation on the user according to the target regulation mode.

Alternatively, the performing an emotion regulation operation on the user includes: directly performing the target regulation mode. In response to the number of the target regulation modes being at least two, at least two target regulation modes may be performed in sequence. For example, in response to the target regulation mode referring to playing music, the electronic device plays music through a music application. In response to the target regulation mode referring to providing a position of an entertainment place or a leisure place near the current position of the vehicle and further automatically navigating to the position, the electronic device searches for the position of the entertainment place or the leisure place near the current position through an electronic map, and automatically activates the navigation functionality of the electronic map to use the current position as the starting point and the position of the entertainment place or the leisure place as the destination point, to obtain a navigation route.

In this embodiment, the database pre-stores the adopted data of the each regulation mode for the to-be-regulated emotion, and the adopted data reflects the degree of acceptance for the regulation mode. Then, the target regulation mode is selected according to the adopted data of the each regulation mode. That is, the regulation mode that is easily accepted by the user is selected. Therefore, according to the regulation mode that is easily accepted by the user, the emotion regulation operation is performed on the user, and thus, the emotion of the user can be effectively regulated, which reduces risks during the driving, and improves the intellectualized degree during the driving.

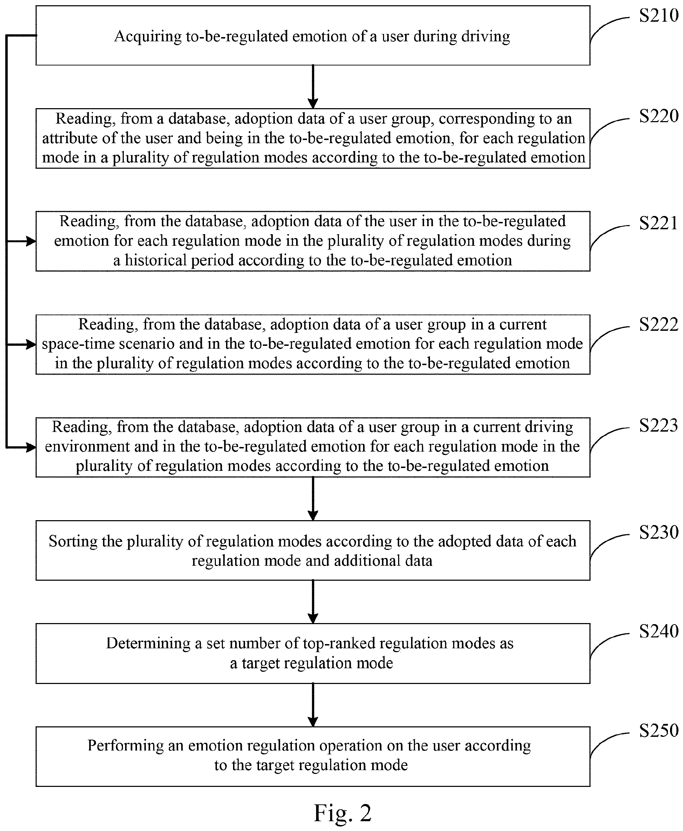

According to an embodiment of the present invention, Fig. 2 is a flowchart of a second method for regulating a user emotion in the embodiment of the present invention. The embodiment of the present invention is optimized on the basis of the technical solution of the above embodiment.

Alternatively, the operation "reading adopted data of each regulation mode in a plurality of regulation modes for the to-be-regulated emotion from a database according to the to-be-regulated emotion" is subdivided into at least one operation of: "reading, from the database, adoption data of a user group, corresponding to an attribute of the user and being in the to-be-regulated emotion, for each regulation mode in the plurality of regulation modes according to the to-be-regulated emotion; reading, from the database, adoption data of the user in the to-be-regulated emotion for the each regulation mode in the plurality of regulation modes during a historical period according to the to-be-regulated emotion; reading, from the database, adoption data of a user group in a current space-time scenario and in the to-be-regulated emotion for each regulation mode in the plurality of regulation modes according to the to-be-regulated emotion; or reading, from the database, adoption data of a user group in a current driving environment and in the to-be-regulated emotion for each regulation mode in the plurality of regulation modes according to the to-be-regulated emotion."

Alternatively, the operation "selecting a target regulation mode from the plurality of regulation modes according to the adopted data of the each regulation mode" is subdivided into: "sorting the plurality of regulation modes according to the adopted data of each regulation mode and additional data; and determining a set number of top-ranked regulation modes as the target regulation mode, the additional data including at least one of: the attribute of the user, the current space-time scenario, the current driving environment, or feature data of the each regulation mode."

The method for regulating a user emotion shown in Fig. 2 includes the following steps.

S210, acquiring to-be-regulated emotion of a user during driving. Continuing to perform at least one of S220, S221, S222 or S223.

S220, reading, from a database, adoption data of a user group, corresponding to an attribute of the user and being in the to-be-regulated emotion, for each regulation mode in a plurality of regulation modes according to the to-be-regulated emotion. Continuing to perform S220.

The attribute of the user includes, but is not limited to, the age, gender and address of the user. Accordingly, the user group corresponding to the attribute of the user includes, but is not limited to, a user group matching the age range of the user, a user group consistent with the gender of the user, and a user group consistent with the address of the user.

Alternatively, before S220, adoption data of a user group, corresponding to at least one attribute and being in each emotion, for each regulation mode is collected. Here, the at least one attribute includes at least one of the age, the gender or the address. Accordingly, the user group includes a group corresponding to a single attribute or a combination of various attributes.

It should be noted that the adopted data is only different from the adoption data in expression, but is essentially the same as the adoption data. Similar to the adopted data, the adoption data also includes at least one of: a number of adoptions, a frequency of adoption, or an adoption rate. The number of adoptions is identical to the number of times that the regulation mode is adopted, the frequency of adoption is identical to the frequency at which the regulation mode is adopted, and the adoption rate is identical to the rate at which the regulation mode is adopted.

Taking that the attribute refers to the age range, and the adoption data refers to the adoption rate as an example, an adoption rate $r_{<a_j, s_n, p_i>}$ of a user group, corresponding to each age range and being in each emotion, for each regulation mode is collected through Equation (1).

$$ r_{<a_j, s_n, p_i>} = \frac{C_{<a_j, s_n, p_i>}}{R_{<a_j, s_n, p_i>}} \ (1). $$

Here, $C_{<a_j, s_n, p_i>}$ represents the number of adoptions of the user group, corresponding to the age range $a_j$ and being in the emotion $s_n$, for the regulation mode $p_i$, and $R_{<a_j, s_n, p_i>}$ represents the number of presentations of the regulation mode $p_i$ to the user group corresponding to the age range $a_j$ and being in the emotion $s_n$.

Next, the adoption data exceeding a set threshold value is stored in the database. The set threshold value may be set autonomously, for example, the set threshold value of the adoption rate is 80%. In this way, a regulation mode with a high adoption rate may be retained, and a regulation mode with a low adoption rate may be filtered out. Therefore, all read from the database are regulation modes having a high adoption rate and the corresponding adoption rate.

S221, reading, from the database, adoption data of the user in the to-be-regulated emotion for each regulation mode in the plurality of regulation modes during a historical period according to the to-be-regulated emotion. Continuing to perform S230.

The historical period may be a period until the current moment, for example, the most recent month or the most recent week.

Alternatively, before S221, adoption data of each user in each emotion for each regulation mode during the historical period is collected, and the adoption data exceeding a set threshold value is stored to the database.

The adoption rate $r_{<u_j,s_n,p_i>}$ of each user in each emotion for each regulation mode during the historical period is collected through Equation (2).

$$r_{<u_j,s_n,p_i>} = \frac{C_{<u_j,s_n,p_i>}}{R_{<u_j,s_n,p_i>}} \ (2).$$

Here, $C_{<u_j,s_n,p_i>}$ represents the number of adoptions of the user $u_j$ in the emotion $s_n$ for the regulation mode $p_i$ during the historical period, and $R_{<u_j,s_n,p_i>}$ represents the number of presentations of the regulation mode $p_i$ to the user $u_j$ in the emotion $s_n$ during the historical period.

Next, the adoption data exceeding the set threshold value is stored in the database. The set threshold value may be set autonomously, for example, the set threshold value of the adoption rate is 80%.

S222, reading, from the database, adoption data of a user group in a current space-time scenario and in the to-be-regulated emotion for each regulation mode in the plurality of regulation modes according to the to-be-regulated emotion. Continuing to perform S230.

The current space-time scenario includes, but is not limited to, a current month, a current time point (e.g., morning, noon and evening), a current holiday, and a current driving destination. The user group in the current space-time scenario includes, but is not limited to, a user group in the current month, a user group at the current time point, a user group in the current holiday, and a user group whose driving destination is the current driving destination. 1

Alternatively, before S222, adoption data of a user group in at least one space-time scenario and in each emotion for each regulation mode is collected, and the adoption data exceeding a set threshold value is stored to the database. Here, the at least one space-time scenario refers to at least one of: the month, the time point, the holiday, or the driving destination. Accordingly, the user group is a group in a single scenario or a combination of various scenarios.

Taking that the space-time scenario refers to the time point and the adoption data refers to the adoption rate as an example, an adoption rate $r_{<t_o,s_n,p_i>}$ of a user group at each time point and in each emotion for each regulation mode is collected through Equation (3).

$$r_{<t_o,s_n,p_i>} = \frac{C_{<t_o,s_n,p_i>}}{R_{<t_o,s_n,p_i>}} \ (3).$$

Here, $C_{<t_o,s_n,p_i>}$ represents the number of adoptions of the user group at the time point $t_o$ and in the emotion $s_n$ for the regulation mode $p_i$, and $R_{<t_o,s_n,p_i>}$ represents the number of presentations of the regulation mode $p_i$ to the user group at the time point $t_o$ and in the

emotion $s_n$.

Next, the adoption data exceeding the set threshold value is stored in the database. The set threshold value may be set autonomously, for example, the set threshold value of the adoption rate is 80%.

S223, reading, from the database, adoption data of a user group in a current driving environment and in the to-be-regulated emotion for each regulation mode in the plurality of regulation modes according to the to-be-regulated emotion. Continuing to perform S230.

The current driving environment includes, but not limited to, a traffic jam environment, a traffic light awaiting environment, and a current vehicle type. The user group in the current driving environment includes, but not limited to, a user group in the traffic jam environment, a user group in the traffic light awaiting environment, and a user group driving a vehicle of the current vehicle type.

Alternatively, before S223, adoption data of a user group in at least one driving environment and in each emotion for each regulation mode is collected, and the adoption data exceeding a set threshold value is stored to the database. Here, the at least one driving environment includes at least one of: the traffic jam environment, the traffic light awaiting environment or the current vehicle type. Accordingly, the user group includes a group in a single driving environment or a combination of various driving environments.

Taking that the driving environment refers to the vehicle type and the adoption data refers to the adoption rate as an example, an adoption rate $r_{<v_j,s_n,p_i>}$ of a user group driving a vehicle of each vehicle type and being in each emotion for each regulation mode is collected through Equation (4).

$$r_{<v_j,s_n,p_i>} = \frac{C_{<v_j,s_n,p_i>}}{R_{<v_j,s_n,p_i>}} \ (4).$$

Here, $C_{<v_j,s_n,p_i>}$ represents the number of adoptions of the user group driving a vehicle of the vehicle type $v_j$ and being in the emotion $s_n$ for the regulation mode $p_i$, and $R_{<v_j,s_n,p_i>}$ represents the number of presentations of the regulation mode $p_i$ to the user group driving the vehicle of the vehicle type $v_j$ and being in the emotion $s_n$.

Next, the adoption data exceeding the set threshold value is stored in the database. The set threshold value may be set autonomously, for example, the set threshold value of the adoption rate is 80%.

S230, sorting the plurality of regulation modes according to the adopted data of each regulation mode and additional data.

The additional data includes at least one of: the attribute of the user, the current space-time scenario, the current driving environment, or feature data of each regulation mode. The feature data of each regulation mode includes, but is not limited to, adoption data of all user groups for each regulation mode, and the type of each regulation mode such as a voice type or a navigation type.

For example, the plurality of regulation modes are scored using a rank function. The rank function is a sorting model obtained through supervised training. In actual application, a GBDT (Gradient Boosting Decision Tree) may be selected. Here, $f(*)$ represents the Rank function, and $S_i$ is the fraction of each regulation mode, as shown in Equation (5).

$$\forall i \ \in C, S_i = f(U_i, A_i, E_i, J_i, H_i, K_i) \ (5).$$

Here, C is the set of the plurality of regulation modes for the to-be-regulated emotion that are read from the database; $U_i$ is the adoption data of the user group corresponding to the attribute of the user and being in the to-be-regulated emotion for each regulation mode in the plurality of regulation modes, the adoption data being read from the database; $A_i$ is the adoption data of the user in the to-be-regulated emotion for each regulation mode in the plurality of regulation modes during the historical period, the adoption data being read from the database; $E_i$ is the adoption data of the user group in the current space-time scenario and in the to-be-regulated emotion for each regulation mode in the plurality of regulation modes, the adoption data being read from the database; $J_i$ is the attribute of the user; $H_i$ is the current space-time scenario and the current driving environment; and $K_i$ is the feature data of each regulation mode.

Then, the plurality of regulation modes are sorted in descending order of scores.

S240, determining a set number of top-ranked regulation modes as a target regulation mode.

Here, the set number is 1, 2 or 3.

S250, performing an emotion regulation operation on the user according to the target regulation mode.

It should be noted that S220, S221, S222 and S223 in Fig. 2 are in a parallel relationship, but are not limited thereto. At least one of S220, S221, S222 or S223 may also be performed in sequence. For example, S220, S221, S222 and S223 may be performed in sequence, and after the performing is completed, S230 is performed.

In this embodiment, the adoption data corresponding to the attribute of the user, the historical period, the current space-time scenario and a current driving scenarios is read, and thus, the degrees of acceptance of users of different attributes in different scenarios for each regulation mode during the historical period are obtained. Then, the regulation mode easily accepted by the user is selected. Therefore, according to the regulation mode easily accepted by the user, the emotion regulation operation is performed on the user, such that the emotion of the user can be effectively regulated, which reduces risks during the driving.

Further, the plurality of regulation modes are sorted according to the adopted data of each regulation mode and additional data. In this way, the regulation mode most likely to be accepted by the user is obtained.

In the above embodiment and the following embodiments, after the operation "reading adopted data of each regulation mode in a plurality of regulation modes for the to-be-regulated emotion from a database according to the to-be-regulated emotion," the operation "in response to there being no adopted data of any regulation mode for the to-be-regulated emotion in the database, or the user being a new user, or a new regulation mode being added, determining the target regulation mode according to a set rule" is added.

Specifically, if there is no adopted data of any regulation mode for the to-be-regulated emotion in the database, or the user is the new user, or the new regulation mode is added, there would be a cold start problem that the adopted data is missing. In order to solve this problem, the target regulation mode is determined according to the set rule. The set rule may be to: manually specify a regulation mode, or randomly select a regulation mode.

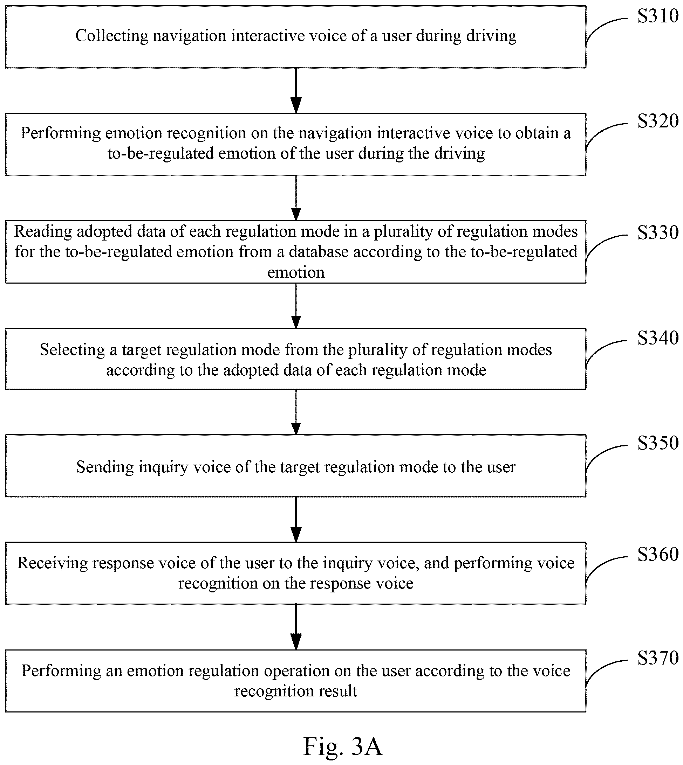

According to an embodiment of the present invention, Fig. 3A is a flowchart of a third method for regulating a user emotion in an embodiment of the present invention. The embodiment of the present invention is optimized on the basis of the technical solutions of the above embodiments.

Alternatively, the operation "acquiring a to-be-regulated emotion of a user during driving" is subdivided into: "collecting navigation interactive voice of the user during the driving; and performing emotion recognition on the navigation interactive voice to obtain the to-be-regulated emotion of the user."

Alternatively, the operation "performing an emotion regulation operation on the user according to the target regulation mode" is subdivided into: "sending inquiry voice of the target regulation mode to the user; receiving response voice of the user to the inquiry voice, and performing voice recognition on the response voice; and performing the emotion regulation operation on the user according to the voice recognition result."

The method for regulating a user emotion shown in Fig. 3A includes the following steps.

S310, collecting navigation interactive voice of a user during driving.

The navigation interactive voice refers to interactive voice sent to an electronic map by the user when using the electronic map to perform navigation, for example, "navigating to a certain address" or "whether there is a traffic jam on the current road section or not." According to this embodiment, the emotion recognition is performed on the navigation interactive voice, and thus, the emotion of the user is effectively regulated in the navigation scenario, thereby reducing risks during the driving.

S320, performing emotion recognition on the navigation interactive voice to obtain a to-be-regulated emotion of the user during the driving.

Alternatively, the emotion of the user is recognized through: 1) an SVM (Support Vector Machine) recognition method based on an MFCC (Mel Frequency Cepstrum Coefficient) voice characteristic; and 2) a convolutional neural network and BILSTM deep neural network recognition method based on original voice characteristics. Here, BILSTM is obtained by combining a forward LSTM (Long Short-Term Memory) and a backward LSTM.

S330, reading adopted data of each regulation mode in a plurality of regulation modes for the to-be-regulated emotion from a database according to the to-be-regulated emotion.

S340, selecting a target regulation mode from the plurality of regulation modes according to the adopted data of each regulation mode.

S350, sending inquiry voice of the target regulation mode to the user.

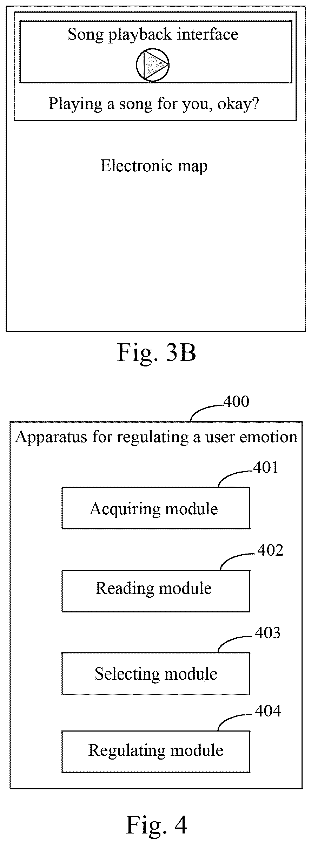

After the target regulation mode is acquired, the user may be inquired for which target regulation mode needs to be performed, and whether the target regulation mode needs to be performed, for example, "playing a song for you or telling a story?" and "playing a song for you, okay?" Fig. 3B is a schematic diagram of an interface of an electronic map in an embodiment of the present invention. The interface of the electronic map displays the text information of the inquiry voice "playing a song for you, okay?" and a song playback interface, and thus, the emotion of the user is regulated in a form of visualization.

S360, receiving response voice of the user to the inquiry voice, and performing voice recognition on the response voice.

After listening to the inquiry voice, the user sends the response voice for the inquiry voice to an electronic device, for example "ok" or "no."

The electronic device performs voice recognition on the response voice to obtain a voice recognition result, which includes yes or no, and may also include a regulation condition, for example, regulation time and a regulation place.

S370, performing an emotion regulation operation on the user according to the voice recognition result.

If the voice recognition result is yes, the target regulation mode is performed. If the voice recognition result is no, the operation is ended. If the voice recognition result is the regulation condition, the target regulation mode is performed according to the regulation condition. As an example, the target regulation mode is performed at the regulation time. As another example, the target regulation mode is performed when driving to the regulation place.

According to this embodiment, the emotion of the user is regulated in an interactive way, such that the personalized requirement of the user can be fulfilled, and the intellectualized degree of the emotion regulation can be improved.

According to an embodiment of the present invention, Fig. 4 is a structural diagram of an apparatus for regulating a user emotion in an embodiment of the present invention. The embodiment of the present invention is applicable to a situation where a regulation mode is selected to automatically regulate an emotion of a user in a driving scenario. The apparatus is implemented by means of software and/or hardware, and specifically configured in an electronic device having a certain data computing capability.

The apparatus 400 for regulating a user emotion shown in Fig. 4 includes an acquiring module 401, a reading module 402, a selecting module 403 and a regulating module 404.

The acquiring module 401 is configured to acquire a to-be-regulated emotion of a user during driving; the reading module 402 is configured to read adopted data of each regulation mode in a plurality of regulation modes for the to-be-regulated emotion from a database according to the to-be-regulated emotion; the selecting module 403 is configured to select a target regulation mode from the plurality of regulation modes according to the adopted data of each regulation mode; and the regulating module 404 is configured to perform an emotion regulation operation on the user according to the target regulation mode.

In this embodiment, the database pre-stores the adopted data of each regulation mode for the to-be-regulated emotion, and the adopted data reflects the degree of acceptance for the regulation mode. Then, the target regulation mode is selected according to the adopted data of each regulation mode. That is, the regulation mode that is easily accepted by the user is selected. Therefore, according to the regulation mode that is easily accepted by the user, the emotion regulation operation is performed on the user, and thus, the emotion of the user can be effectively regulated, which reduces risks during the driving.

Further, the reading module includes at least one unit of: an attribute unit, configured to read, from the database, adoption data of a user group, corresponding to an attribute of the user and being in the to-be-regulated emotion, for each regulation mode in the plurality of regulation modes according to the to-be-regulated emotion; a historical period unit, configured to read, from the database, adoption data of the user in the to-be-regulated emotion for each regulation mode in the plurality of regulation modes during a historical period according to the to-be-regulated emotion; a space-time scenario unit, configured to read, from the database, adoption data of a user group in a current space-time scenario and in the to-be-regulated emotion for each regulation mode in the plurality of regulation modes according to the to-be-regulated emotion; or a driving environment unit, configured to read, from the database, adoption data of a user group in a current driving environment and in the to-be-regulated emotion for each regulation mode in the plurality of regulation modes according to the to-be-regulated emotion.

Further, the apparatus further includes at least one module of: an attribute collecting module, configured to collect adoption data of a user group, corresponding to at least one attribute and being in each emotion, for each regulation mode, and store adoption data exceeding a set threshold value to the database; a historical period collecting module, configured to collect adoption data of each user in each emotion for each regulation mode during the historical period, and store adoption data exceeding the set threshold value to the database; a space-time scenario collecting module, configured to collect adoption data of a user group in at least one space-time scenario and in each emotion for each regulation mode, and store adoption data exceeding the set threshold value to the database; or a driving environment collecting module, configured to collect adoption data of a user group in at least one driving environment and in each emotion for each regulation mode, and store adoption data exceeding the set threshold value to the database.

Further, the adopted data includes at least one of: a number of times that the regulation mode is adopted, a frequency at which the regulation mode is adopted, or a rate at which the regulation mode is adopted.

Further, the selecting module includes: a sorting unit, configured to sort the plurality of regulation modes according to the adopted data of each regulation mode and additional data; and a determining unit, configured to determine a set number of top-ranked regulation modes as the target regulation mode. Here, the additional data includes at least one of: the attribute of the user, the current space-time scenario, the current driving environment, or feature data of each regulation mode.

Further, the apparatus further includes a set rule regulation module, configured to determine the target regulation mode according to a set rule, in response to there being no adopted data of any regulation mode for the to-be-regulated emotion in the database, or the user being a new user, or a new regulation mode being added.

Further, the acquiring module includes: a collecting unit, configured to collect navigation interactive voice of the user during the driving; and a recognizing unit, configured to perform emotion recognition on the navigation interactive voice to obtain the to-be-regulated emotion of the user during the driving.

Further, the regulating module 404 is specifically configured to send inquiry voice of the target regulation mode to the user; receive response voice of the user to the inquiry voice, and perform voice recognition on the response voice; and perform the emotion regulation operation on the user according to the voice recognition result.

The apparatus for regulating a user emotion may perform the method for regulating a user emotion provided in any embodiment of the present invention, and possess functional modules for performing the method for regulating a user emotion, and corresponding beneficial effects.

According to an embodiment of the present invention, the present invention further provides an electronic device and a readable storage medium.

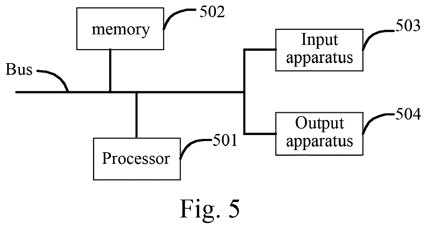

As shown in Fig. 5, which is a block diagram of an electronic device of a method for regulating a user emotion according to an embodiment of the present invention. The electronic device is intended to represent various forms of digital computers, such as laptop computers, desktop computers, workbenches, personal digital assistants, servers, blade servers, mainframe computers, and other suitable computers. The electronic device may also represent various forms of mobile apparatuses, such as personal digital processing, cellular phones, smart phones, wearable devices, and other similar computing apparatuses. The components shown herein, their connections and relationships, and their functions are merely examples, and are not intended to limit the implementation of the present invention described and/or claimed herein.

As shown in Fig. 5, the electronic device includes: one or more processors 501, a memory 502, and interfaces for connecting various components, including high-speed interfaces and low-speed interfaces. The various components are connected to each other using different buses, and may be installed on a common motherboard or in other methods as needed. The processor may process instructions executed within the electronic device, including instructions stored in or on the memory to display graphic information of GUI on an external input/output apparatus (such as a display device coupled to the interface). In other embodiments, a plurality of processors and/or a plurality of buses may be used together with a plurality of memories if desired. Similarly, a plurality of electronic devices may be connected, and the devices provide some necessary operations (for example, as a server array, a set of blade servers, or a multi-processor system). In Fig. 5, one processor 601 is used as an example.

The memory 502 is a non-transitory computer readable storage medium provided by the present invention. The memory stores instructions executable by at least one processor, so that the at least one processor performs the method for regulating a user emotion provided by the present invention. The non-transitory computer readable storage medium of the present invention stores computer instructions for causing a computer to perform the method for regulating a user emotion provided by the present invention.

The memory 502, as a non-transitory computer readable storage medium, may be used to store non-transitory software programs, non-transitory computer executable programs and modules, such as program instructions/modules corresponding to the method for regulating a user emotion in the embodiments of the present invention (for example, the acquiring module 401, reading module 402, selecting module 403 and regulating module 404 shown in Fig. 4). The processor 501 executes the non-transitory software programs, instructions, and modules stored in the memory 502 to execute various functional applications and data processing of the server, that is, to implement the method for regulating a user emotion in the foregoing method embodiment.

The memory 502 may include a storage program area and a storage data area, where the storage program area may store an operating system and at least one functionality required application program; and the storage data area may store data created by the use of the electronic device according to the method for regulating a user emotion, etc. In addition, the memory 502 may include a high-speed random access memory, and may also include a non-transitory memory, such as at least one magnetic disk storage device, a flash memory device, or other non-transitory solid-state storage devices. In some embodiments, the memory 502 may optionally include memories remotely provided with respect to the processor 501, and these remote memories may be connected to the electronic device of the method for regulating a user emotion through a network. Examples of the above network include but are not limited to the Internet, intranet, local area network, mobile communication network, and combinations thereof.

The electronic device of the method for regulating a user emotion may further include: an input apparatus 503 and an output apparatus 504. The processor 501, the memory 502, the input apparatus 503, and the output apparatus 504 may be connected through a bus or in other methods. In Fig. 5, connection through a bus is used as an example.

The input apparatus 503 may receive input digital or character information, and generate key signal inputs related to user settings and functionality control of the electronic device of the method for regulating a user emotion, such as touch screen, keypad, mouse, trackpad, touchpad, pointing stick, one or more mouse buttons, trackball, joystick and other input apparatuses. The output apparatus 504 may include a display device, an auxiliary lighting apparatus (for example, LED), a tactile feedback apparatus (for example, a vibration motor), and the like. The display device may include, but is not limited to, a liquid crystal display (LCD), a light emitting diode (LED) display, and a plasma display. In some embodiments, the display device may be a touch screen.

Various embodiments of the systems and technologies described herein may be implemented in digital electronic circuit systems, integrated circuit systems, dedicated ASICs (application specific integrated circuits), computer hardware, firmware, software, and/or combinations thereof. These various embodiments may include: being implemented in one or more computer programs that can be executed and/or interpreted on a programmable system that includes at least one programmable processor. The programmable processor may be a dedicated or general-purpose programmable processor, and may receive data and instructions from a storage system, at least one input apparatus, and at least one output apparatus, and transmit the data and instructions to the storage system, the at least one input apparatus, and the at least one output apparatus.

These computing programs (also referred to as programs, software, software applications, or codes) include machine instructions of the programmable processor and may use high-level processes and/or object-oriented programming languages, and/or assembly/machine languages to implement these computing programs. As used herein, the terms "machine readable medium" and "computer readable medium" refer to any computer program product, device, and/or apparatus (for example, magnetic disk, optical disk, memory, programmable logic apparatus (PLD)) used to provide machine instructions and/or data to the programmable processor, including machine readable medium that receives machine instructions as machine readable signals. The term "machine readable signal" refers to any signal used to provide machine instructions and/or data to the programmable processor.

In order to provide interaction with a user, the systems and technologies described herein may be implemented on a computer, the computer has: a display apparatus for displaying information to the user (for example, CRT (cathode ray tube) or LCD (liquid crystal display) monitor); and a keyboard and a pointing apparatus (for example, mouse or trackball), and the user may use the keyboard and the pointing apparatus to provide input to the computer. Other types of apparatuses may also be used to provide interaction with the user; for example, feedback provided to the user may be any form of sensory feedback (for example, visual feedback, auditory feedback, or tactile feedback); and any form (including acoustic input, voice input, or tactile input) may be used to receive input from the user.

The systems and technologies described herein may be implemented in a computing system that includes backend components (e.g., as a data server), or a computing system that includes middleware components (e.g., application server), or a computing system that includes frontend components (for example, a user computer having a graphical user interface or a web browser, through which the user may interact with the implementations of the systems and the technologies described herein), or a computing system that includes any combination of such backend components, middleware components, or frontend components. The components of the system may be interconnected by any form or medium of digital data communication (e.g., communication network). Examples of the communication network include: local area networks (LAN), wide area networks (WAN), the Internet, and blockchain networks.

The computer system may include a client and a server. The client and the server are generally far from each other and usually interact through the communication network. The relationship between the client and the server is generated by computer programs that run on the corresponding computer and have a client-server relationship with each other.

An embodiment of the present invention further provides a computer program product, including a computer program. The computer program, when executed by a processor, causes the processor to implement the method for recognizing a word slot according any embodiment of the present invention.

**Claims**

1. A method for regulating a user emotion, comprising:

    acquiring (51 10) a to-be-regulated emotion of a user during driving;
    reading (S120) adopted data of each regulation mode in a plurality of regulation modes for the to-be-regulated emotion from a database according to the to-be-regulated emotion;
    selecting (S130) a target regulation mode from the plurality of regulation modes according to the adopted data of the each regulation mode; and
    performing (S140) an emotion regulation operation on the user according to the target regulation mode,
    wherein the adopted data comprises at least one of: a number of times that each regulation mode is adopted, a frequency at which each regulation mode is adopted, or a rate at which each regulation mode is adopted.

2. The method according to claim 1, wherein the reading adopted data of each regulation mode in a plurality of regulation modes for the to-be-regulated emotion from a database according to the to-be-regulated emotion comprises at least one operation of:

    reading, from the database, adoption data of a user group, corresponding to an attribute of the user and being in the to-be-regulated emotion, for each regulation mode in the plurality of regulation modes according to the to-be-regulated emotion;
    reading, from the database, adoption data of the user in the to-be-regulated emotion for each regulation mode in the plurality of regulation modes during a historical period according to the to-be-regulated emotion;
    reading, from the database, adoption data of a user group in a current space-time scenario and in the to-be-regulated emotion for each regulation mode in the plurality of regulation modes according to the to-be-regulated emotion; or
    reading, from the database, adoption data of a user group in a current driving environment and in the to-be-regulated emotion for the each regulation mode in the plurality of regulation modes according to the to-be-regulated emotion.

3. The method according to claim 2, wherein before the reading adopted data of each regulation mode in a plurality of regulation modes for the to-be-regulated emotion from a database according to the to-be-regulated emotion, the method further comprises at least one operation of:

    collecting adoption data of a user group, corresponding to at least one attribute and being in each emotion, for each regulation mode, and storing adoption data exceeding a set threshold value to the database;
    collecting adoption data of the caetr user in the each emotion for each regulation mode during the historical period, and storing adoption data exceeding the set threshold value to the database;
    collecting adoption data of a user group in at least one space-time scenario and in the each emotion for the each regulation mode, and storing adoption data exceeding the set threshold value to the database; or
    collecting adoption data of a user group in at least one driving environment and in each emotion for each regulation mode, and storing adoption data exceeding the set threshold value to the database.

4. The method according to claim 2, wherein the selecting a target regulation mode from the plurality of regulation modes according to the adopted data of each regulation mode comprises:

    sorting the plurality of regulation modes according to the adopted data of each regulation mode and additional data; and
    determining a set number of top-ranked regulation modes as the target regulation mode,
    wherein the additional data comprises at least one of: the attribute of the user, the current space-time scenario, the current driving environment, or feature data of each regulation mode.

5. The method according to any one of claims 1-4, wherein after the reading adopted data of each regulation mode in a plurality of regulation modes for the to-be-regulated emotion from a database according to the to-be-regulated emotion, the method further comprises:
in response to there being no adopted data of any regulation mode for the to-be-regulated emotion in the database, or the user being a new user, or a new regulation mode being added, determining the target regulation mode according to a set rule.

6. The method according to any one of claims 1-5, wherein the acquiring a to-be-regulated emotion of a user during driving comprises:

collecting navigation interactive voice of the user during the driving; and
performing emotion recognition on the navigation interactive voice to obtain the to-be-regulated emotion of the user during the driving.

7. The method according to any one of claims 1-6, wherein the performing an emotion regulation operation on the user according to the target regulation mode comprises:

sending inquiry voice of the target regulation mode to the user;
receiving response voice of the user to the inquiry voice, and performing voice recognition on the response voice; and
performing the emotion regulation operation on the user according to the voice recognition result.

8. An apparatus for regulating a user emotion, comprising:

an acquiring module (401), configured to acquire a to-be-regulated emotion of a user during driving;
a reading module (402), configured to read adopted data of each regulation mode in a plurality of regulation modes for the to-be-regulated emotion from a database according to the to-be-regulated emotion;
a selecting module (403), configured to select a target regulation mode from the plurality of regulation modes according to the adopted data of the each regulation mode; and
a regulating module (404), configured to perform an emotion regulation operation on the user according to the target regulation mode,
wherein the adopted data comprises at least one of: a number of times that each regulation mode is adopted, a frequency at which each regulation mode is adopted, or a rate at which each regulation mode is adopted.

9. The apparatus according to claim 8, wherein the reading module (402) comprises at least one unit of:

an attribute unit, configured to read, from the database, adoption data of a user group, corresponding to an attribute of the user and being in the to-be-regulated emotion, for each regulation mode in the plurality of regulation modes according to the to-be-regulated emotion;
a historical period unit, configured to read, from the database, adoption data of the user in the to-be-regulated emotion for each regulation mode in the plurality of regulation modes during a historical period according to the to-be-regulated emotion;
a space-time scenario unit, configured to read, from the database, adoption data of a user group in a current space-time scenario and in the to-be-regulated emotion for each regulation mode in the plurality of regulation modes according to the to-be-regulated emotion; or
a driving environment unit, configured to read, from the database, adoption data of a user group in a current driving environment and in the to-be-regulated emotion for each regulation mode in the plurality of regulation modes according to the to-be-regulated emotion.

10. The apparatus according to claim 9, wherein the selecting module (403) comprises:

a sorting unit, configured to sort the plurality of regulation modes according to the adopted data of each regulation mode and additional data; and
a determining unit, configured to determine a set number of top-ranked regulation modes as the target regulation mode,
wherein the additional data comprises at least one of: the attribute of the user, the current space-time scenario, the current driving environment, or feature data of each regulation mode.

11. The apparatus according to any one of claims 8-10, wherein the acquiring module (401) comprises:

a collecting unit, configured to collect navigation interactive voice of the user during the driving; and
a recognizing unit, configured to perform emotion recognition on the navigation interactive voice to obtain the to-be-regulated emotion of the user during the driving.

12. A non-transitory computer readable storage medium, storing computer instructions, wherein the computer instructions are configured to cause the apparatus according to any one of claims 8-11 to perform the method for regulating a user emotion according to any one of claims 1-7.

**Patentansprüche**

1. Verfahren zum Regulieren einer Benutzer-Emotion, umfassend:

Erfassen (S110) einer zu regulierenden Emotion eines Benutzers während des Fahrens;

Lesen (S120) von übernommenen Daten für jeden Regulationsmodus aus einer Vielzahl von Regulationsmodi für die zu regulierende Emotion aus einer Datenbank gemäß der zu regulierenden Emotion;

Auswählen (S130) eines Ziel-Regulationsmodus aus der Vielzahl von Regulationsmodi gemäß den übernommenen Daten jedes Regulationsmodus; und

Durchführen (S140) eines Emotionsregulationsvorgangs an dem Benutzer gemäß dem Zielregulationsmodus,

wobei die übernommenen Daten zumindest eines der folgenden umfasst: eine Anzahl von Malen, die jeder Regulationsmodus übernommen wird, eine Häufigkeit, mit der jeder Regulationsmodus übernommen wird, oder eine Rate, mit der jeder Regulationsmodus übernommen wird.

2. Verfahren nach Anspruch 1, wobei das Lesen übernommener Daten jedes Regulationsmodus aus einer Vielzahl von Regulationsmodi für die zu regulierende Emotion aus einer Datenbank gemäß der zu regulierenden Emotion zumindest einen Vorgang der folgenden umfasst:

Lesen von Übernahmedaten einer Benutzergruppe, die einem Attribut des Benutzers entsprechen und in der zu regulierenden Emotion sind, aus der Datenbank für jeden Regulationsmodus aus der Vielzahl von Regulationsmodi gemäß der zu regulierenden Emotion;

Lesen von Übernahmedaten des Benutzers in der zu regulierenden Emotion für jeden Regulationsmodus aus der Vielzahl von Regulationsmodi während eines historischen Zeitraums gemäß der zu regulierenden Emotion aus der Datenbank;

Lesen von Übernahmedaten einer Benutzergruppe in einem aktuellen Raum-Zeit-Szenario und in der zu regulierenden Emotion für jeden Regulationsmodus aus der Vielzahl von Regulationsmodi gemäß der zu regulierenden Emotion aus der Datenbank; oder

Lesen von Übernahmedaten einer Benutzergruppe in einer aktuellen Fahrumgebung und in der zu regulierenden Emotion für jeden Regulationsmodus aus der Vielzahl von Regulationsmodi gemäß der zu regulierenden Emotion aus der Datenbank.

3. Verfahren nach Anspruch 2, wobei vor dem Lesen von übernommenen Daten jedes Regulationsmodus aus einer Vielzahl von Regulationsmodi für die zu regulierende Emotion aus einer Datenbank gemäß der zu regulierenden Emotion, wobei das Verfahren ferner zumindest einen Vorgang der folgenden umfasst:

Sammeln von Übernahmedaten einer Benutzergruppe, entsprechend zumindest einem Attribut und die in jeder Emotion vorliegen, für jeden Regulationsmodus und Speichern von Übernahmedaten, die einen eingestellten Schwellenwert überschreiten, in der Datenbank;

Sammeln von Übernahmedaten des Benutzers in jeder der Emotion für jeden Regulationsmodus während des historischen Zeitraums und Speichern von Übernahmedaten, die den eingestellten Schwellenwert überschreiten, in der Datenbank;

Sammeln von Übernahmedaten einer Benutzergruppe in zumindest einem Raum-Zeit-Szenario und in jeder der Emotionen für jeden Regulationsmodus und Speichern von Übernahmedaten, die den eingestellten Schwellenwert überschreiten, in die Datenbank; oder

Sammeln von Übernahmedaten einer Benutzergruppe in zumindest einer Fahrumgebung und jeder Emotion für jeden Regulationsmodus und Speichern von Übernahmedaten, die den eingestellten Schwellenwert überschreiten, in der Datenbank.

4. Verfahren nach Anspruch 2, wobei das Auswählen eines Ziel-Regulationsmodus aus der Vielzahl von Regulationsmodi gemäß den übernommenen Daten jedes Regulationsmodus Folgendes umfasst:

Sortieren der Vielzahl von Regulationsmodi gemäß den übernommenen Daten jedes Regulationsmodus und zusätzlichen Daten; und

Bestimmen einer eingestellten Anzahl von am höchsten gereihten Regulationsmodi als Ziel-Regulationsmodus,

wobei die zusätzlichen Daten zumindest eines der folgenden umfassen: das Attribut des Benutzers, das aktuelle Raum-Zeit-Szenario, die aktuelle Fahrumgebung oder Merkmalsdaten jedes Regulationsmodus.

5. Verfahren nach einem der Ansprüche 1 bis 4, wobei nach dem Lesen übernommener Daten jedes Regulationsmodus aus einer Vielzahl von Regulationsmodi für die zu regulierende Emotion aus einer Datenbank gemäß der zu regulierenden Emotion, das Verfahren ferner Folgendes umfasst:

als Reaktion darauf, dass keine übernommenen Daten keines Regulationsmodus für die zu regulierende Emotion in der Datenbank vorliegen oder dass der Benutzer ein neuer Benutzer ist oder ein neuer Regulationsmodus hinzugefügt wird, Bestimmen, des Ziel-Regulationsmodus gemäß einer festgelegten Regel.

**6.** Verfahren nach einem der Ansprüche 1 bis 5, wobei das Erfassen einer zu regulierenden Emotion eines Benutzers während des Fahrens Folgendes umfasst:

Sammeln einer interaktiven Navigationsstimme des Benutzers während des Fahrens; und Durchführen einer Emotionserkennung der interaktiven Navigationsstimme, um eine zu regulierende Emotion des Benutzers während des Fahrens zu erhalten.

**7.** Verfahren nach einem der Ansprüche 1 bis 6, wobei das Durchführen eines Emotionsregulierungsvorgangs des Benutzers gemäß dem Ziel-Regulationsmodus Folgendes umfasst:

Senden einer Abfragestimme des Ziel-Regulationsmodus an den Benutzer; Empfangen einer Antwortstimme des Benutzers auf die Abfragestimme und Durchführen einer Stimmerkennung der Antwortstimme; und Durchführen des Emotionsregulationsvorgangs des Benutzers gemäß des Stimmerkennungsergebnisses.

**8.** Vorrichtung zum Regulieren einer Benutzeremotion, umfassend:

ein Erfassungsmodul (401), das konfiguriert ist, um eine zu regulierende Emotion eines Benutzers während des Fahrens zu erfassen; ein Lesemodul (402), das konfiguriert ist, um übernommene Daten jedes Regulationsmodus aus einer Vielzahl von Regulationsmodi für die zu regulierende Emotion aus einer Datenbank gemäß der zu regulierenden Emotion zu lesen; ein Auswahlmodul (403), das konfiguriert ist, um einen Ziel-Regulationsmodus aus der Vielzahl von Regulationsmodi gemäß den übernommenen Daten jedes Regulationsmodus auszuwählen; und ein Regulationsmodul (404), das konfiguriert ist, um einen Emotionsregulierungsvorgang des Benutzers gemäß dem Ziel-Regulationsmodus durchzuführen, wobei die übernommenen Daten zumindest eines der folgenden umfassen: eine Anzahl von Malen, die jeder Regulationsmodus übernommen wird, eine Häufigkeit, mit der jeder Regulationsmodus übernommen wird oder eine Rate, mit der jeder Regulationsmodus übernommen wird.

**9.** Vorrichtung nach Anspruch 8, wobei das Lesemodul (402) zumindest eine Einheit aus Folgenden umfasst:

eine Attributeinheit, die konfiguriert ist, um Übernahmedaten einer Benutzergruppe, die einem Attribut des Benutzers entsprechen und in der zu regulierenden Emotion vorliegen, für jeden Regulationsmodus aus der Vielzahl von Regulationsmodi gemäß der zu regulierenden Emotion aus der Datenbank zu lesen; eine historische Zeitraumeinheit, die konfiguriert ist, um Übernahmedaten des Benutzers in der zu regulierenden Emotion für jeden Regulationsmodus aus der Vielzahl von Regulationsmodi während eines historischen Zeitraums gemäß der zu regulierenden Emotion aus der Datenbank zu lesen; eine Raum-Zeit-Szenario-Einheit, die konfiguriert ist, um Übernahmedaten einer Benutzergruppe in einem aktuellen Raum-Zeit-Szenario und in der zu regulierenden Emotion für jeden Regulationsmodus aus der Vielzahl von Regulationsmodi gemäß der zu regulierenden Emotion aus der Datenbank zu lesen; oder eine Fahrumgebungseinheit, die konfiguriert ist, um Übernahmedaten einer Benutzergruppe in einer aktuellen Fahrumgebung und in der zu regulierenden Emotion für jeden Regulationsmodus aus der Vielzahl von Regulationsmodi gemäß der zu regulierenden Emotion aus der Datenbank zu lesen.

**10.** Vorrichtung nach Anspruch 9, wobei das Auswahlmodul (403) Folgendes umfasst:

eine Sortiereinheit, die konfiguriert ist, um die Vielzahl von Regulationsmodi gemäß den übernommenen Daten jedes Regulationsmodus und zusätzlichen Daten zu sortieren; und eine Bestimmungseinheit, die konfiguriert ist, um eine festgelegte Anzahl von am höchsten gereihten Regulationsmodi als Ziel-Regulationsmodus zu bestimmen, wobei die zusätzlichen Daten zumindest eines der folgenden umfasst: das Attribut des Benutzers, das aktuelle Raum-Zeit-Szenario, die aktuelle Fahrumgebung oder Merkmalsdaten jedes Regulationsmodus.

**11.** Vorrichtung nach einem der Ansprüche 8 bis 10, wobei das Erfassungsmodul (401) Folgendes umfasst:

eine Sammeleinheit, die konfiguriert ist, um eine interaktive Navigationsstimme des Benutzers während der Fahrt zu sammeln; und eine Erkennungseinheit, die konfiguriert ist, um eine Emotionserkennung auf der interaktiven Navigationsstimme durchzuführen, um die zu regulierende Emotion des Benutzers während der Fahrt zu erhalten.

**12.** Nichtflüchtiges computerlesbares Speichermedium, das Computerbefehle speichert, wobei die Computerbefehle konfiguriert sind, um zu bewirken, dass eine Vorrichtung nach einem der Ansprüche 8 bis 11 ein Verfahren zum Regulieren einer Benutzeremotion nach einem der Ansprüche 1 bis 7 durchführt.

**Revendications**

**1.** Procédé de régulation d'une émotion d'utilisateur, comprenant les étapes consistant à :

acquérir (S110) une émotion devant être régulée d'un utilisateur pendant qu'il conduit ;
lire (S120) des données adoptées de chaque mode de régulation dans une pluralité de modes de régulation pour l'émotion devant être régulée à partir d'une base de données selon l'émotion devant être régulée ;
sélectionner (S130) un mode de régulation cible parmi la pluralité de modes de régulation selon les données adoptées de chaque mode de régulation ; et
effectuer (S140) une opération de régulation d'émotion sur l'utilisateur selon le mode de régulation cible,
dans lequel les données adoptées comprennent au moins un parmi : un nombre de fois où chaque mode de régulation est adopté, une fréquence à laquelle chaque mode de régulation est adopté, ou un rythme auquel chaque mode de régulation est adopté.

**2.** Procédé selon la revendication 1, dans lequel la lecture de données adoptées de chaque mode de régulation dans une pluralité de modes de régulation pour l'émotion devant être régulée à partir d'une base de données selon l'émotion devant être régulée comprend au moins une opération consistant à :

lire, à partir de la base de données, des données d'adoption d'un groupe d'utilisateurs, correspondant à un attribut de l'utilisateur et se trouvant dans l'émotion devant être régulée, pour chaque mode de régulation dans la pluralité de modes de régulation selon l'émotion devant être régulée ;
lire, à partir de la base de données, des données d'adoption de l'utilisateur dans l'émotion devant être régulée pour chaque mode de régulation dans la pluralité de modes de régulation pendant une période historique selon l'émotion devant être régulée ;
lire, à partir de la base de données, des données d'adoption d'un groupe d'utilisateurs dans un scénario spatio-temporel actuel et dans l'émotion devant être régulée pour chaque mode de régulation dans la pluralité de modes de régulation selon l'émotion devant être régulée ; ou
lire, à partir de la base de données, des données d'adoption d'un groupe d'utilisateurs dans un environnement de conduite actuel et dans l'émotion devant être régulée pour chaque mode de régulation dans la pluralité de modes de régulation selon l'émotion devant être régulée.

**3.** Procédé selon la revendication 2, dans lequel avant la lecture de données adoptées de chaque mode de régulation dans une pluralité de modes de régulation pour l'émotion devant être régulée à partir d'une base de données selon l'émotion devant être régulée, le procédé comprend en outre au moins une opération consistant à :

collecter des données d'adoption d'un groupe d'utilisateurs, correspondant à au moins un attribut et étant dans chaque émotion, pour chaque mode de régulation, et stocker des données d'adoption dépassant une valeur de seuil définie dans la base de données ;
collecter des données d'adoption de l'utilisateur dans chaque émotion pour chaque mode de régulation pendant la période historique, et stocker des données d'adoption dépassant la valeur de seuil définie dans la base de données ;
collecter des données d'adoption d'un groupe d'utilisateurs dans au moins un scénario spatio-temporel et dans chaque émotion pour chaque mode de régulation, et stocker des données d'adoption dépassant la valeur de seuil définie dans la base de données ; ou
collecter des données d'adoption d'un groupe d'utilisateurs dans au moins un environnement de conduite et dans chaque émotion pour chaque mode de régulation, et stocker des données d'adoption dépassant la valeur de seuil définie dans la base de données.

**4.** Procédé selon la revendication 2, dans lequel la sélection d'un mode de régulation cible parmi la pluralité de modes de régulation selon les données adoptées de chaque mode de régulation comprend les étapes consistant à :

trier la pluralité de modes de régulation selon les données adoptées de chaque mode de régulation et des données supplémentaires ; et
déterminer un nombre défini de modes de régulation de premier rang en tant que mode de régulation cible,
dans lequel les données supplémentaires comprennent au moins un parmi : l'attribut de l'utilisateur, le scénario spatio-temporel actuel, l'environnement de conduite actuel, ou des données de caractéristique de chaque mode de régulation.

5. Procédé selon l'une quelconque des revendications 1 à 4, dans lequel, après la lecture de données adoptées de chaque mode de régulation dans une pluralité de modes de régulation pour l'émotion devant être régulée à partir d'une base de données selon l'émotion devant être régulée, le procédé comprend en outre l'étape consistant à :

en réponse à l'absence de données adoptées d'un mode de régulation quelconque pour l'émotion devant être régulée dans la base de données, ou à l'utilisateur étant un nouvel utilisateur, ou à un nouveau mode de régulation étant ajouté, déterminer le mode de régulation cible selon une règle établie.

6. Procédé selon l'une quelconque des revendications 1 à 5, dans lequel l'acquisition d'une émotion devant être régulée d'un utilisateur qui conduit comprend les étapes consistant :

collecter une voix interactive de navigation de l'utilisateur pendant qu'il conduit ; et effectuer une reconnaissance d'émotion sur la voix interactive de navigation pour obtenir l'émotion devant être régulée de l'utilisateur pendant qu'il conduit.

7. Procédé selon l'une quelconque des revendications 1 à 6, dans lequel l'exécution d'une opération de régulation d'émotion sur l'utilisateur selon le mode de régulation cible comprend les étapes consistant à : envoyer à l'utilisateur une voix d'interrogation du mode de régulation cible ;

réceptionner une voix de réponse de l'utilisateur à la voix d'interrogation, et exécuter une reconnaissance vocale sur la voix de réponse ; et effectuer l'opération de régulation d'émotion sur l'utilisateur selon le résultat de reconnaissance vocale.

8. Appareil pour réguler une émotion d'utilisateur, comprenant :

un module d'acquisition (401), configuré pour acquérir une émotion devant être régulée d'un utilisateur pendant qu'il conduit ; un module de lecture (402), configuré pour lire des données adoptées de chaque mode de régulation dans une pluralité de modes de régulation pour l'émotion devant être régulée à partir d'une base de données selon l'émotion devant être régulée ; un module de sélection (403), configuré pour sélectionner un mode de régulation cible parmi la pluralité de modes de régulation selon les données adoptées de chaque mode de régulation ; et un module de régulation (404), configuré pour effectuer une opération de régulation d'émotion sur l'utilisateur selon le mode de régulation cible, dans lequel les données adoptées comprennent au moins un parmi : un nombre de fois où chaque mode de régulation est adopté, une fréquence à laquelle chaque mode de régulation est adopté, ou un rythme auquel chaque mode de régulation est adopté.

9. Appareil selon la revendication 8, dans lequel le module de lecture (402) comprend au moins une unité parmi :

une unité d'attribut, configurée pour lire, à partir de la base de données, des données d'adoption d'un groupe d'utilisateurs, correspondant à un attribut de l'utilisateur et se trouvant dans l'émotion devant être régulée, pour chaque mode de régulation dans la pluralité de modes de régulation selon l'émotion devant être régulée ; une unité de période historique, configurée pour lire, à partir de la base de données, des données d'adoption de l'utilisateur dans l'émotion devant être régulée pour chaque mode de régulation dans la pluralité de modes de régulation pendant une période historique selon l'émotion devant être régulée ; une unité de scénario spatio-temporel, configurée pour lire, à partir de la base de données, des données d'adoption d'un groupe d'utilisateurs dans un scénario spatio-temporel actuel et dans l'émotion devant être régulée pour chaque mode de régulation dans la pluralité de modes de régulation selon l'émotion devant être régulée ; ou une unité d'environnement de conduite, configurée pour lire, à partir de la base de données, des données d'adoption d'un groupe d'utilisateurs dans un environnement de conduite actuel et dans l'émotion devant être régulée pour chaque mode de régulation dans la pluralité de modes de régulation selon l'émotion devant être régulée.

10. Appareil selon la revendication 9, dans lequel le module de sélection (403) comprend :

une unité de tri, configurée pour trier la pluralité de modes de régulation selon les données adoptées de chaque mode de régulation et des données supplémentaires ; et une unité de détermination, configurée pour déterminer un nombre défini de modes de régulation de premier rang en tant que mode de régulation cible, dans lequel les données supplémentaires comprennent au moins un parmi : l'attribut de l'utilisateur, le scénario spatio-temporel actuel, l'environnement de conduite actuel, ou des données de caractéristique de chaque mode de régulation.

11. Appareil selon l'une quelconque des revendications 8 à 10, dans lequel le module d'acquisition (401) comprend :

   une unité de collecte, configurée pour collecter une voix interactive de navigation de l'utilisateur pendant qu'il conduit ; et
   une unité de reconnaissance, configurée pour effectuer une reconnaissance d'émotion sur la voix interactive de navigation afin d'obtenir l'émotion devant être régulée de l'utilisateur pendant qu'il conduit.

12. Support de stockage non transitoire lisible par ordinateur, stockant des instructions informatiques, dans lequel les instructions informatiques sont configurées pour amener l'appareil selon l'une quelconque des revendications 8 à 11 à exécuter le procédé de régulation d'une émotion d'utilisateur selon l'une quelconque des revendications 1 à 7.

**EP 3 831 636 B1**

Acquiring a to-be-regulated emotion of a user during driving — S110

Reading adopted data of each regulation mode in a plurality of regulation modes for the to-be-regulated emotion from a database according to the to-be-regulated emotion — S120

Selecting a target regulation mode from the plurality of regulation modes according to the adopted data of each regulation mode — S130

Performing an emotion regulation operation on the user according to the target regulation mode — S140

Fig. 1

18

Acquiring to-be-regulated emotion of a user during driving — S210

Reading, from a database, adoption data of a user group, corresponding to an attribute of the user and being in the to-be-regulated emotion, for each regulation mode in a plurality of regulation modes according to the to-be-regulated emotion — S220

Reading, from the database, adoption data of the user in the to-be-regulated emotion for each regulation mode in the plurality of regulation modes during a historical period according to the to-be-regulated emotion — S221

Reading, from the database, adoption data of a user group in a current space-time scenario and in the to-be-regulated emotion for each regulation mode in the plurality of regulation modes according to the to-be-regulated emotion — S222

Reading, from the database, adoption data of a user group in a current driving environment and in the to-be-regulated emotion for each regulation mode in the plurality of regulation modes according to the to-be-regulated emotion — S223

Sorting the plurality of regulation modes according to the adopted data of each regulation mode and additional data — S230

Determining a set number of top-ranked regulation modes as a target regulation mode — S240

Performing an emotion regulation operation on the user according to the target regulation mode — S250

Fig. 2

Collecting navigation interactive voice of a user during driving — S310

Performing emotion recognition on the navigation interactive voice to obtain a to-be-regulated emotion of the user during the driving — S320

Reading adopted data of each regulation mode in a plurality of regulation modes for the to-be-regulated emotion from a database according to the to-be-regulated emotion — S330

Selecting a target regulation mode from the plurality of regulation modes according to the adopted data of each regulation mode — S340

Sending inquiry voice of the target regulation mode to the user — S350

Receiving response voice of the user to the inquiry voice, and performing voice recognition on the response voice — S360

Performing an emotion regulation operation on the user according to the voice recognition result — S370

Fig. 3A

Song playback interface

Playing a song for you, okay?

Electronic map

Fig. 3B

400

Apparatus for regulating a user emotion

401
Acquiring module

402
Reading module

403
Selecting module

404
Regulating module

Fig. 4

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20110102058 A

- CN 106803423 A